# EUROPÄISCHE PATENTSCHRIFT

(11) **EP 2 956 427 B1**
(45) Veröffentlichungstag und Bekanntmachung des Hinweises auf die Patenterteilung: **10.04.2019**
(21) Anmeldenummer: 14704766.6
(22) Anmeldetag: 07.02.2014
(51) Int. Cl.: C04B 35/486, C04B 35/488, A61C 13/083, A61L 27/02, A61C 8/00, A61K 6/02, A61K 6/00, A61C 13/00

(54) **MEHRPHASIGE WERKSTOFFE AUF BASIS VON ZIRKONOXID**
ZIRCONIUM OXIDE-BASED MULTI-PHASE MATERIALS
MATÉRIAUX POLYPHASES À BASE D'OXYDE DE ZIRCONIUM

(30) Priorität: 13.02.2013 DE 102013202287
(43) Veröffentlichungstag der Anmeldung: 23.12.2015
(73) Patentinhaber: CeramTec GmbH, 73207 Plochingen (DE)
(72) Erfinder: KUNTZ, Meinhard, 73733 Esslingen (DE); FRIEDERICH, Kilian, 73207 Plochingen (DE); GOTTWIK, Lukas, 73092 Heiningen (DE); MORHARDT, Andreas, 73730 Esslingen (DE); BROSI, Juliane, 70372 Stuttgart (DE)
(74) Vertreter: Fehrenbacher, Eckhard Anton
(86) Internationale Anmeldenummer: PCT/EP2014/052407
(87) Internationale Veröffentlichungsnummer: WO 2014/124874

(56) Entgegenhaltungen:
- EP-A1- 2 377 506
- WO-A1-90/11980
- WO-A1-2008/040813
- WO-A1-2014/029757
- MIURA M ET AL: "FORMATION OF PLATE-LIKE LANTHANUM-B-ALUMINATE CRYSTAL IN CE-TZP MATRIX", JOURNAL OF MATERIALS SCIENCE, KLUWER ACADEMIC PUBLISHERS, DORDRECHT, Bd. 29, Nr. 1, 1. Januar 1994 (1994-01-01) , Seiten 262-268, XP000433025, ISSN: 0022-2461, DOI: 10.1007/BF00356602

## Beschreibung

Die Erfindung betrifft die Herstellung und die Verwendung von mehrphasigen Werkstoffen auf Basis von Zirkonoxid. Insbesondere betrifft die Erfindung polykristalline Werkstoffe auf Basis von Zirkonoxid sowie Sinterformkörper aus diesem Material, die im medizinischen Bereich beispielsweise als Implantate oder Zahnersatz Verwendung finden können.

Die Zirkonoxidkeramik ist biokompatibel und kann mit herkömmlichen und standardisierten Aufbereitungsmethoden hergestellt werden. Die mechanischen Eigenschaften und die hydrothermale Alterungsbeständigkeit sind hinsichtlich einer schädigungsfreien Hartbearbeitbarkeit und des verwendeten Einsatzgebiets- bzw. Milieus angepasst. Das übergeordnete Anwendungsgebiet der Zirkonoxidkeramik ist im Bereich der Biokeramik. Untergeordnete Anwendungsfelder sind zum Beispiel die Zahnprothetik (Blanks, Brücken und Kronen), Zahnimplantate, Abutments, und Wirbelsäulenimplantate (Cage, Spacer) aber auch allgemein Anwendungsfelder, in denen eine technische Keramik mit schädigungsfreier Hartbearbeitbarkeit benötigt wird, wie z.B. beim maschinellen Bearbeiten wie Schleifen, Fräsen und Bohren.

Keramische Werkstoffe besitzen aufgrund ihrer chemischen Beständigkeit, ihrer mechanischen, physikalischen und insbesondere optischen Eigenschaften, die eine exzellente Ästhetik zulassen, im dentalen Markt Vorteile gegenüber herkömmlichen metallischen Werkstoffen.

Der Allgemeine Trend bei Dentalkeramiken geht in Richtung "vollkeramische Systeme". Dennoch werden heute noch häufig Keramiken als Verblendungen auf metallischen Gerüsten aufgebracht.

Dentalkeramiken lassen sich anhand ihrer Herstellungsmethode und ihrer kristallinen Phase klassifizieren. Unterschieden wird grundsätzlich in Metall-Keramische Systeme und in vollkeramische Systeme.

Metall-Keramische Systeme gibt es seit 1960. Um eine ästhetisch-akzeptable Restauration in Anlehnung an den natürlichen Zahn zu erhalten, wird eine Verblendkeramik auf ein Metallgerüst aufgebracht. Typische Verblendmaterialien bestehen aus feldspatischen Gläsern, gewöhnlich Leuzitkristall-basiert. Die Zugabe von Leuzitkristallen (KAlSi₂O₆) in das feldspatische Glasgefüge führt zu optimalen Eigenschaften hinsichtlich der thermischen Ausdehnungskoeffizienten von Gerüst und Verblendung. Leuzitkristalle bilden sich durch inkongruentes Schmelzen von natürlichem Feldspat bei Temperaturen zwischen 1150 und 1530°C. Durch die Variation des Leuzitkristallgehalts im Glas kann der thermische Ausdehnungskoeffizient gezielt gesteuert und an das metallische Gerüst angepasst werden. Gewöhnlich beträgt der typische Leuzitkristallgehalt in feldspatischem Glas zwischen 15 und 25 Vol-%. Damit ist der thermische Ausdehnungskoeffizient geringer als der des Metalls, wodurch die aufgebrachte Verblendung nach der Abkühlung unter Druck gesetzt ist.

Klassisch werden Verblendkeramiken unter Vakuum gesintert, um die Porosität im Endprodukt zu reduzieren. Die mechanischen Eigenschaften, insbesondere die Festigkeit und Risszähigkeit der Leuzitkristall-basierten Gläser (auch Dentalporzellan genannt) sind aufgrund der Glasphase die geringsten von allen keramischen Werkstoffen, die in der Zahnmedizin verwendet werden. Bis 2005 wurden noch 50% aller Zahnrestaurationen mit Metall-Keramischen Systemen hergestellt.

Vollkeramische Systeme sind metallfrei und seit 30 Jahren verfügbar. Die Prozesstechnik wird ständig weiterentwickelt (z.B. Heißpressen, Schlickergießen, CAD/CAM Bearbeitung). Der Hauptunterschied zu den Metall-Keramischen Systemen ist ein weit höherer kristalliner Phasenanteil zwischen 35 und 100 Vol-%. Die mechanischen Eigenschaften verbessern sich, aber auch die Opazität nimmt zu, was hinsichtlich der geforderten Ästhetik nachteilig ist. Es gibt eine Vielzahl von Faktoren, die auf die Langlebigkeit der Vollkeramischen Systeme einen Einfluss haben, z.B. orales Umgebungsmilieu, schwankende pH-Werte von sauer bis basisch, zyklische Belastung oder extreme Belastungsspitzen während des Kauens. Vollkeramische Systeme mit höherem Glasphasenanteil zeigen häufig Spannungsrisskorrosion als Versagensursache. Aufgrund hydrothermaler Alterung von Y-TZP-Keramiken (Yttria-stabilized Tetragonal Zirconia Polycrystal mit 100 Vol-% kristalliner Phase) bei niedrigen Temperaturen werden Tests in Normen verlangt, bei denen die Langlebigkeit in menschlicher Umgebung und unter zyklischer Belastung bewertet werden soll.

Vollkeramische Systeme werden hauptsächlich aufgrund der Herstellungsmethode klassifiziert (z.B. Heißpressen, Trockenpressen und Sintern, Schlickergießen, CAD/CAM Bearbeitung).

Beim Heißpressen wurden zuerst Leuzitkristall-basierte Gläser mit einem kristallinen Phasenanteil zwischen 35 und 45 Vol-% verwendet. Die Festigkeit der Leuzitkristall-basierten Gläser beträgt ca. 150 MPa und ist damit etwa um den Faktor 2 höher als bei Leuzitkristall-basierten Gläsern der Metall-Keramischen Systeme. Mehrmaliges Aufheizen kann die Leuzitkristallisation begünstigen und zu höheren Festigkeiten führen.

Heute wird eine neue Glaskeramik für das Heißpressen verwendet. Das Material besteht aus einem Lithiumdisilikat-basierten Glas mit einem kristallinen Phasenanteil von 65 Vol-%. Röntgenographische Untersuchungen haben neben Lithiumdisilikat (Li₂Si₂O₅) weitere Kristallphasen wie Lithiummetasilikat (Li₂SiO₃) und Cristobalit (SiO₂) gezeigt. Die Festigkeit ist im Vergleich zu den Leuzitkristall-basierten Gläsern um ca. den Faktor 2 höher und liegt bei ca. 250 MPa.

Das Trockenpressen und Sintern von vollkeramischen Systemen wird seit den frühen 90iger Jahren verwendet. Die Herstellung erfolgt computerunterstützt und berücksichtigt die Sinterschwindung des Presslings beim Sintern. Als Gerüstmaterial werden Aluminiumoxid- und Zirkonoxid-basierte Keramiken (100 Vol-% kristalliner Phasenanteil) verwendet, wobei auf das Gerüstmaterial zusätzlich eine Verblendung aus Glaskeramik aufgebracht wird. Aluminiumoxidkeramiken zeichnen sich durch eine Biegefestigkeit von ca. 600 MPa und ein exzellentes In-vivo Verhalten aus.

Das Schlickergießen wird seit den 90iger Jahren angewendet. Dabei wird ein poröser Grünkörper mittels Schlickerguss aus kristallinen Phasen hergestellt, anschließend gesintert und mit einem Lanthan-basiertem Glas infiltriert. Auf dem Dentalmarkt sind folgende Glaskeramiken erhältlich: Aluminiumoxid (Al₂O), Spinell (MgAl₂O₄) oder 12Ce-TZP/Al₂O₃-Komposit. Das glasinfiltrierte Aluminiumoxid hat vergleichbare mechanische Eigenschaften zu Lithiumdisilikat-basierter Glaskeramik, jedoch eine minimal höhere Opazität. Der glasinfiltrierte Spinell besitzt deutlich mehr Transluzenz und vergleichbare mechanische Eigenschaften zur Lithiumdisilikat-basierten Glaskeramik. Das glasinfiltrierte Zirkonoxid/Aluminiumoxid-Komposit zeigt die höchsten Festigkeiten und Risszähigkeiten aller schlickergegossenen Dentalkeramiken.

Die computergesteuerte CAD/CAM Bearbeitung von keramischen Blöcken bzw. Blanks erfolgt seit den frühen 70iger Jahren und wurde von Duret eingeführt. Damals erfolgte die Bearbeitung an dichtgesinterten Blanks. Heute wird hauptsächlich mit vorgesinterten Blanks gearbeitet.

Glaskeramik eignet sich für die CAD/CAM-Bearbeitung im dichtgesinterten Zustand aufgrund der sehr guten Bearbeitbarkeit. Früher wurden typische Glimmerkristallbasierte Gläser aufgrund der idealen Bearbeitbarkeit verwendet. Heute werden feldspatische Gläser mit Sanidin-, Leuzit- oder Lithiumdisilikatkristallen verwendet. Allerdings zeigt die CAD/CAM-Bearbeitung an dichtgesinterten Glaskeramiken einen signifikanten Werkzeugverschleiß. Oberflächenfehler können das In-vivo Verhalten negativ beeinflussen.

Allgemein sind Glaskeramiken sehr gut bearbeitbar. Aufgrund unterschiedlicher thermischer Ausdehnungskoeffizienten von Kristall und Glasmatrix entstehen jedoch während des Abkühlens Mikrorisse entlang der Phasengrenzen. Zusätzlich besitzen die kristallinen Phasen eine sehr gute Spaltbarkeit entlang der Längsausrichtung (vor allem Glimmer entlang der kristallographischen (001)-Ebene. Die Kristallphasen sollten daher keine Vorzugsorientierung im Glasgefüge haben. Ein durch ein Werkzeug eingebrachter Riss verläuft entlang von Spaltebenen oder auch entlang von Phasengrenzen zwischen Kristall und Glasmatrix. Dadurch wird der Riss während der Bearbeitung ständig abgelenkt und es werden nur kleine Bereiche der Oberfläche aus dem Werksstück herausgebrochen. Dieser Verstärkungsmechanismus ist auch unter dem Begriff Rissablenkung bekannt.

Seit 2001 erfolgt die CAD-CAM-Bearbeitung an vorgesinterten Zirkonoxid-Blanks. Die Bearbeitung ist leichter, schneller und zeigt einen geringeren Werkzeugverschleiß gegenüber der Hartbearbeitung an dicht-gesinterten Zirkonoxid-Blanks. Jedoch müssen die gefertigten Werkstücke anschließend dicht gesintert werden. Schwankungen in der Sinterschwindung einhergehend mit Maßabweichungen, sowie Nachbesserungsarbeiten per Hand vom Zahntechniker führen zu einem erhöhten Schädigungsrisiko des Zirkonoxids. Fast alle erhältlichen Zirkonoxid-Blanks werden aus Tosoh-Rohstoff gefertigt. Zirkonoxid als Gerüstmaterial besitzt bisher die besten mechanischen Eigenschaften. Es treten aber häufig aufgrund der zusätzlich benötigten Verblendkeramik am Interface zwischen Gerüst und Verblendung Risse durch Phasenumwandlung der tetragonalen Zirkonoxidphase auf. Es gibt bereits seit einiger Zeit mehrere, bereits veröffentliche 3-Jahres- und 5-Jahres-Invivo-Studien. Das Fazit der Studien ist eine exzellent Erfolgsrate, aber mit geringer Überlebensrate bei Komplikationen wie z.B. Kariesbefall oder Abplatzungen der Verblendung. Der aktuelle Entwicklungstrend geht eindeutig Richtung Zirkonoxid/Aluminiumoxid-Verbundwerkstoffe mit den Zielen, die hydrothermale Alterungsbeständigkeit und mechanischen Eigenschaften zu verbessern.

Die Dokumente EP 2 377 506 A1, WO 2008/040813 A1, WO 90/11980 A1 und WO 2014/029757 A1, sowie der Artikel "Formation of plate-like lanthanum-β-Aluminate crystal in Ce-TZP matrix" (M. Miura et al., JOURNAL OF MATERIALS SCIENCE, vol. 29, no. 1, 1994, pages 262-268), offenbaren Zirkonoxid-basierte Verbundwerkstoffe.

Aufgabe der Erfindung ist daher die Bereitstellung eines verbesserten keramischen Werkstoffs auf Basis von Zirkonoxid, insbesondere für den Bereich der Dentalkeramik, der gute mechanische Eigenschaften mit einer geringeren Härte sowie verbesserten Schädigungstoleranz vereinbart und mittels gängiger Verfahren verarbeitet werden kann.

Die Aufgabe wird durch einen Werkstoff und einen Sinterformkörper gemäß den unabhängigen Ansprüchen gelöst.

Im Rahmen dieser Erfindung ist mit dem Begriff "Werkstoff" eine fertig gesinterte Keramik gemeint. Die beschriebenen Zusammensetzungen beziehen sich also, wenn nichts anderes definiert ist, auf einen keramischen Sinterkörper.

Die Erfindung betrifft mehrphasige keramische Werkstoffe auf Basis von tetragonalem Zirkonoxid. Die tetragonale Phase des Zirkonoxids wird durch Verwendung von Oxiden der Seltenen Erden Samariumoxid (Sm₂O₃) und Gadoliniumoxid (Gd₂O₃) als Additive stabilisiert. Die tetragonale Zirkonoxidphase ist die Hauptkomponente und im Werkstoff grundsätzlich mit einem Volumenanteil von 94-96% vertreten.

Ein erfindungsgemäßer Zirkonoxid-Werkstoff enthält zwischen 4 und 6 Vol.-% eines thermodynamisch stabilen Aluminats. Ein solcher Werkstoff wird im Rahmen dieser Erfindung als "Verbundwerkstoff" bezeichnet.

In einer besonders bevorzugten Ausführungsform der Erfindung beträgt der Volumenanteil des Zirkonoxids etwa 95 Vol.-% am Gesamtwerkstoff-Volumen. Eine zweite Hauptkomponente besteht aus einem thermodynamisch stabilen Aluminat, bevorzugt Strontiumaluminat oder Lanthanaluminat, mit einem Volumenanteil von etwa 5 Vol.-%. Die zweite Hauptkomponente besteht zu über 80 Vol.-% aus Strontiumaluminat oder Lanthanaluminat.

Es hat sich überraschend gezeigt, dass sich die erfindungsgemäßen Werkstoffrezepturen besonders für eine schädigungsarme Hartbearbeitung eignen, das heißt, die Werkstoffeigenschaften werden auch bei ungünstigen Bearbeitungsbedingungen kaum beeinträchtigt.

Die Herstellung eines erfindungsgemäßen Sinterformkörpers aus dem erfindungsgemäßen Zirkonoxidwerkstoff erfolgt mittels an sich bekannter, konventioneller Keramiktechnologie. Die wesentlichen Prozessschritte sind beispielsweise:
a) Pulvermischung gemäß vorgegebener Zusammensetzung in Wasser ansetzen; ggf. Verwendung von Verflüssigern zur Vermeidung der Sedimentation;
b) Homogenisierung im Dissolver (schnelllaufender Rührer);
c) Mahlen in Rührwerkskugelmühle, dabei Erhöhung der spezifischen Oberfläche der Pulvermischung (=Zerkleinerung und Homogenisierung);
d) Evtl. Zugabe von organischen Bindern;
e) Sprühtrocknen, dabei entsteht ein rieselfähiges Granulat mit definierten Eigenschaften;
f) Befeuchten des Granulats mit Wasser und ggf. weiteren Presshilfsmitteln;
g) Axialpressen, Isostatisches Pressen von Blöcken oder endkonturnahe Formgebung mit keramischer Spritzgusstechnologie;
h) Spanabhebende Bearbeitung von Blöcken im Grün- oder vorgesinterten Zustand, dabei wird unter Berücksichtigung der Sinterschwindung weitgehend die Endkontur abgebildet;
i) Sinterung (Diese kann auch in einer 3-stufigen Sinterung erfolgen: 1. Vorbrand auf eine theoretische Dichte von ca. 97%. Die noch verbleibenden Restporen sind nach außen geschlossen. 2. Heißisostatisches Pressen unter hoher Temperatur und hohem Gasdruck, dadurch praktisch vollständige Endverdichtung. 3. So genannter Weißbrand, dadurch wird das beim heißisostatischen Pressen erzeugte Ungleichgewicht der Sauerstoffionen in der Keramik ausgeglichen.);
j) Hartbearbeitung durch Schleifen und Polieren mit diamantbesetztem Werkzeug.

Verwendet werden kann der erfindungsgemäße Zirkonoxidwerkstoff beispielsweise zur Herstellung von Sinterformkörpern, zur Herstellung von künstlichem Zahnersatz, Zahnrestaurationen wie Brücken, Kronen, Inlays und Onlays, zur Herstellung von Zahnwurzelstiften, Implantaten, Abutments, Cages und Spacer im Wirbelsäulenbereich sowie von unikondylären und bikondylären Kniekomponenten. Bevorzugt wird die Anwendung im Bereich des künstlichen Zahnersatzes und der Zahnrestaurationen. Besonders bevorzugt ist die Anwendung im molaren Zahnbereich.

Der Anteil an chemischen Stabilisatoren im erfindungsgemäßen Zirkonoxidwerkstoff (Anteil jeweils relativ zum Zirkonoxidgehalt) beträgt für Sm₂O₃ und Gd₂O₃ 1 bis 5 Mol-%, bevorzugt 2,5 - 3,5 Mol-%. Der Gesamtanteil an chemischen Stabilisatoren im erfindungsgemäßen Zirkonoxidwerkstoff, umfassend einen oder mehrere Zuschlagstoffe, d.h. chemische Stabilisatoren, wobei als chemische Stabilisatoren Sm₂O₃ und Gd₂O₃ oder Mischungen aus diesen Oxiden enthalten sind.

Der Gesamtgehalt an chemischen Stabilisatoren ist < 15 Mol-%, bevorzugt < 14 Mol-%.

Bei der Verwendung von CeO₂ als chemischem Stabilisator besitzt das Zirkonoxid eine Gefügekorngröße von durchschnittlich 0,5 bis 1,5 µm, bevorzugt von durchschnittlich 0,5 bis 1,0 µm. Es hat sich überraschend gezeigt, dass die Gefügekorngröße durch die Verwendung von Gd₂O₃ und Sm₂O₃ erheblich reduziert werden kann. Dabei liegen die Gefügekorngrößen bevorzugt zwischen 0,1 und 0,3 µm, besonders bevorzugt zwischen 0,1 bis 0,2 µm. Daher weist ein Sinterformkörper gemäß einer bevorzugten Ausführungsform Zirkonoxidkristalle mit einer durchschnittlichen Größe zwischen 0,1 und 1,5 µm, bevorzugt von zwischen 0,1 und 0,4 µm und besonders bevorzugt zwischen 0,1 und 0,3 µm auf.

Gemäß einer weiteren Ausführungsform der Erfindung kann das Zirkonoxid zusätzlich lösliche Bestandteile enthalten. Lösliche Bestandteile können z.B. Cr, Fe, Mg, Ca, Ti, Y, Ce, Lanthaniden und/oder V sein. Diese Bestandteile können zum einen als Farbadditive und zum anderen als Sinterhilfmittel fungieren. Die löslichen Bestandteile können in das Kristallgitter eingebaut, d.h. substituiert, werden oder in Form von Verbindungen, z.B. in Mischkristallen, in der Korngrenzphase abgeschieden sein.

Die Bruchfestigkeit eines Sinterformkörpers aus dem erfindungsgemäßen Zirkonoxidwerkstoff ist vorzugsweise ≥ 500 MPa, besonders bevorzugt ≥ 800 MPa.

Es hat sich überraschend gezeigt, dass die Art und Menge des chemischen Stabilisators einen deutlichen Einfluss auf die Härte des Zirkonoxidwerkstoffs hat und auch die Risszähigkeit beeinflusst.

Die Vorteile des erfindungsgemäßen neuen Werkstoffs gegenüber dem Stand der Technik werden anhand der verbesserten "Schädigungstoleranz" quantitativ erfasst. Schädigungstoleranz ist ein mechanischer Kennwert, der den Widerstand eines Werkstoffs gegen eine von außen aufgebrachte Schädigung beschreibt. Die Schädigung kann in der Praxis beispielsweise durch eine Schleifbearbeitung mit diamantbesetzten Werkzeugen erfolgen.

Für die Messung der Schädigungstoleranz im Labor wird auf den Prüfkörper mittels einer Diamantspitze (Vickers) unter einer definierten Beanspruchungskraft eine Schädigung aufgebracht. Im Bereich des Härteeindrucks bilden sich Risse aus, so dass der Prüfkörper an dieser Stelle eine Schwächung erfährt. Die Schwächung wird quantitativ durch Messung der Rest-Bruchspannung, bzw. Restfestigkeit an dieser Stelle ermittelt. Je höher die Restfestigkeit nach einer definierten Schwächung, um so höher ist die Schädigungstoleranz des Werkstoffs.

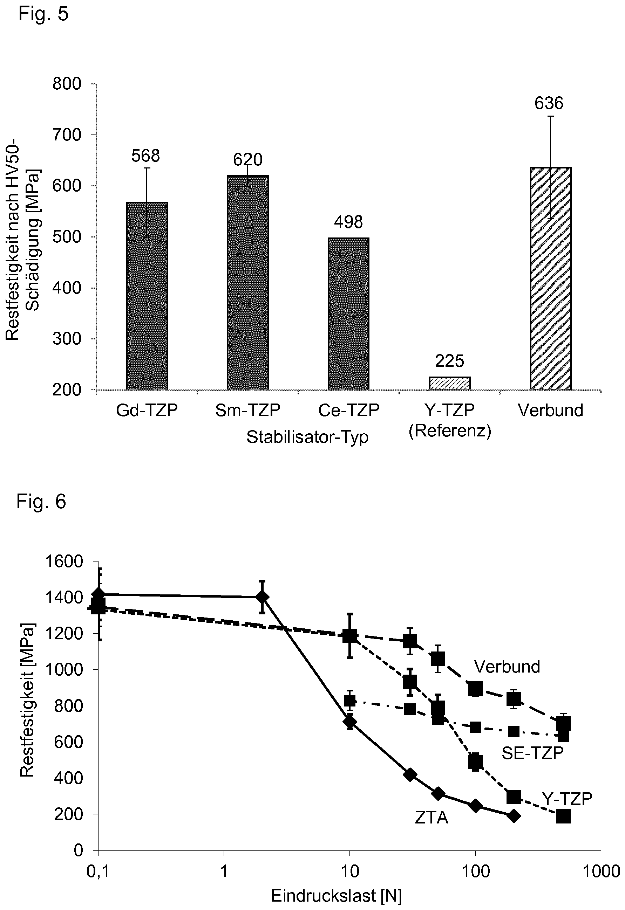

Zur detaillierten Beschreibung der Schädigungstoleranz werden an einer Serie von Prüfkörpern Schädigungen mit unterschiedlichen Beanspruchungskräften aufgebracht. Dadurch ergibt sich eine Kennlinie für den Werkstoff (Restfestigkeit vs. Beanspruchungskraft). Der Nachweis einer verbesserten Schädigungstoleranz eines Werkstoffs gegenüber dem Stand der Technik erfolgt durch Vergleich dieser Kennlinien, siehe Figs. 5 und 6.

Es hat sich überraschend gezeigt, dass die Schädigungstoleranz des Zirkonoxidwerkstoffs durch die Art des chemischen Stabilisators beeinflusst wird.

Nachfolgend werden diese Erkenntnisse anhand von Figuren und Versuchsreihen näher erläutert, ohne sie dadurch einzuschränken:
Die Figuren zeigen:
- Fig. 1:: Diagramm, das die Härte von Sinterformkörpern aus Zirkonoxid in Abhängigkeit vom verwendeten chemischen Stabilisator zeigt
- Fig. 2:: Diagramm, das die Risszähigkeit von Sinterformkörpern aus Zirkonoxid in Abhängigkeit vom verwendeten chemischen Stabilisator zeigt
- Fig. 3:: Gefügekorngröße in Abhängigkeit vom verwendeten chemischen Stabilisator
- Fig. 4:: Gefügebilder in Abhängigkeit vom verwendeten chemischen Stabilisator
- Fig. 5:: Restfestigkeiten nach HV50-Schädigung in Abhängigkeit vom verwendeten chemischen Stabilisator
- Fig. 6:: Schädigungstoleranz-Kennlinien von erfindungsgemäßen Zirkonoxidwerkstoff, erfindungsgemäßen Verbundwerkstoff und Referenz Y-TZP
- Fig. 7:: Hydrothermale Alterungsbeständigkeit in Abhängigkeit vom verwendeten chemischen Stabilisator

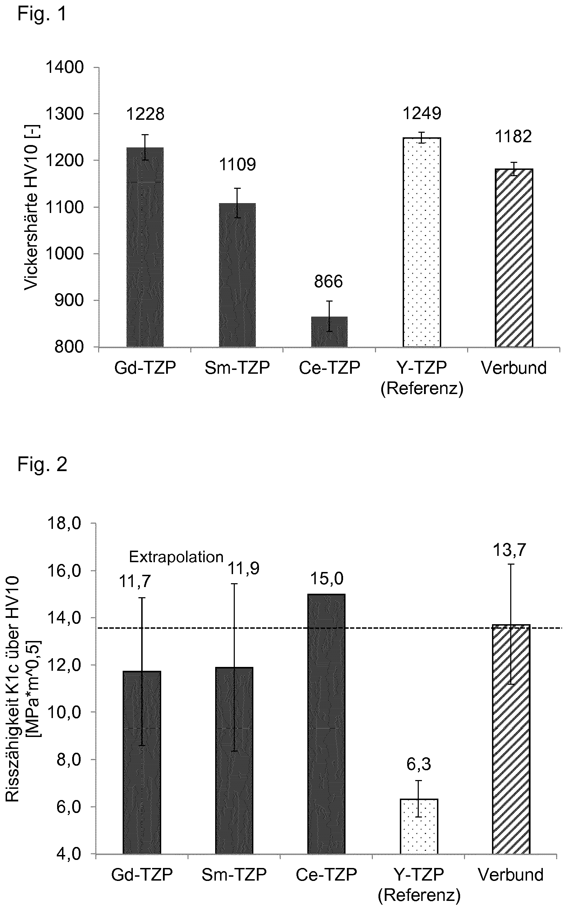

### Versuchsreihe 1: Härte in Abhängigkeit des chemischen Stabilisators (Fig. 1)

Figur 1 zeigt die Ergebnisse einer Versuchsreihe mit erfindungsgemäßen chemischen Stabilisatoren. Getestet wurden die chemischen Stabilisatoren Yttriumoxid (Y₂O₃), Ceroxid (CeO₂), Samariumoxid (Sm₂O₃) und Gadoliniumoxid (Gd₂O₃) sowie ein erfindungsgemäßer Verbundwerkstoff aus Strontiumhexaaluminatverstärktem Zirkonoxid (strontiumhexaaluminate toughened zirconia). Dabei hat sich überraschend gezeigt, dass die Variante mit Ce-Stabilisierung deutlich geringere Härtewerte im Vergleich zur Y-Stabilisierung aufweist. Samarium- und Gadoliniumoxid senken die Härte nur geringfügig, im Falle von Samariumoxid jedoch signifikant. Die Härte wurde mittels Vickerseindruck (HV10) mit einer Kraft von 98,07 N ermittelt.

Im Hinblick auf die erfindungsgemäße Anwendung im dentalen Bereich sind geringere Härten erwünscht. Im molaren Zahnbereich kann ein künstlicher Zahnersatz aus häufig verwendetem Y-TZP auf einen natürlichen Zahn treffen. Die Härte von Y-TZP liegt bei ca. 1250 (HV10). Der natürliche Zahn bzw. das Enamel besitzt aufgrund der eingelagerten Hydroxylapatitkristalle eine deutlich geringere Härte von ca. 400 (HV10). Diese Härtedifferenz kann z.B. bei einer stressbedingten Knirschbewegung (Bruxsimus) zu einem erheblichen Abrieb des natürlichen Zahns führen. Des Weiteren begünstigt eine geringere Härte des Zirkonoxidwerkstoffs eine schädigungsfreie Hartbearbeitung. Daher umfasst eine weitere bevorzugte Ausführungsform der Erfindung einen Zirkonoxidwerkstoff, der Stabilisatoren enthält, die die Härte des Zikronoxidwerkstoffs senken, wobei die Härte eines aus dem Zirkonoxidwerkstoff hergestellten Sinterkörpers kleiner als 1250 (HV10), bevorzugt kleiner als 900 (HV 10) ist.

### Versuchsreihe 2: Risszähigkeit in Abhängigkeit des chemischen Stabilisators (Fig. 2)

Figur 2 zeigt eine Versuchsreihe, die den Einfluss des chemischen Stabilisators auf die Risszähigkeit des Zirkonoxidwerkstoffs darstellt. Es konnte überraschend gezeigt werden, dass die Verwendung von Ceroxid (CeO₂), Samariumoxid (Sm₂O₃) und Gadoliniumoxid (Gd₂O₃) als chemische Stabilisatoren die Risszähigkeit deutlich erhöht. Die Risszähigkeit der erfindungsgemäßen Varianten wurde am Vickershärteeindruck (HV10) ermittelt. Die nicht erfindungsgemäßen Varianten mit CeO₂-Stabilisierung zeigten keine Risse am Härteeindruck. Die erfindungsgemäßen Varianten mit Sm₂O₃- und Gd₂O₃-Stabilisierung zeigten keine oder nur geringfügige Risse am Härteeindruck. Die Varianten, die keine Risse am Härteeindruck zeigen, sind hochzähe Zirkonoxidwerkstoffe. Ihre Risszähigkeit wurde mittels Extrapolation auf 15 MPa*m^0,5 geschätzt. Der Bereich der extrapolierten Werte ist in Fig.2 oberhalb einer gestrichtelten Linie dargestellt und betrifft Werte oberhalb von 13,4 MPa*m^0,5. Dieser Wert ist der höchste, gemessene Risszähigkeitswert, der mit dieser Bestimmungsmethode gemessen wurde.

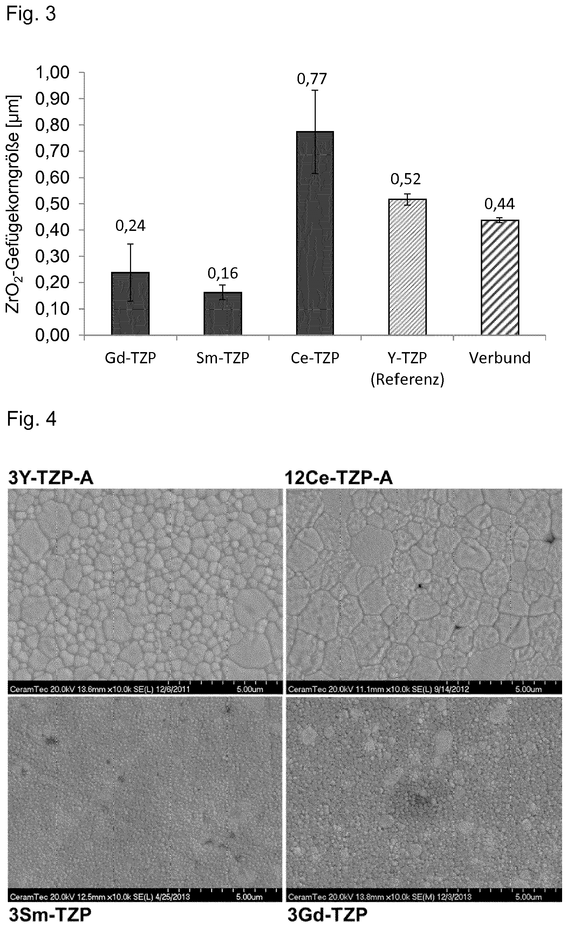

### Versuchsreihe 3: Gefügekorngröße und Gefügebilder in Abhängigkeit des chemischen Stabilisators (Figs. 3, und 4)

In den Figs. 3 und 4 ist der Einfluss des chemischen Stabilisators auf die Gefügekorngröße des erfindungsgemäßen Zirkonoxidwerkstoffs zu sehen. Die Gefügebewertung erfolgte am Rasterelektronenmikroskop. Die Korngrößenermittlung erfolge nach dem Linienschnittverfahren zur Bestimmung der "mittleren Schnittlängen-Korngröße" einer Gefügephase. Es hat sich überraschend gezeigt, dass durch die Verwendung von Gadolinium- und Samariumoxid das Werkstoffgefüge verfeinert werden kann. Die Verwendung von Samariumoxid führte zu einer mittleren Gefügekorngröße von 0,16 µm. Die Verwendung von Gadoliniumoxid führte zu einer mittleren Gefügekorngröße von 0,24 µm. Die erfindungsgemäße Zirkonoxidvariante mit Gd₂O₃-Stabilisierung zeigt eine lokale Grobkornbildung im Gefügebild, siehe Fig. 4. Die einzelnen Grobkörner liegen in der kubischen Zirkonoxidphase vor, welche die Transluzenz des erfindungsgemäßen Werkstoffs geringfügig gegenüber des Dentalstandards Y-TZP begünstigen.

### Versuchsreihe 4: Schädigungstoleranz in Abhängigkeit vom chemischen Stabilisatoren (Fig. 5)

Figur 5 zeigt erfindungsgemäße Zirkonoxidwerkstoffe mit unterschiedlichen Stabilisatoren. Auf der x-Achse sind die verschiedenen Werkstoffe anhand ihrer verwendeten Stabilisatoren aufgezeigt. Auf der y-Achse wurde die Restfestigkeit der erfindungsgemäßen Werkstoffe nach HV50-Schädigung in MPa abgetragen.

Es zeigt sich deutlich, dass im Falle der erfindungsgemäßen Zirkonoxid- und Verbundwerkstoffe die Restfestigkeitswerte im Vergleich zum Referenzwerkstoff bzw. Dentalstandard Y-TZP um ein Vielfaches ansteigen.

### Versuchreihe 5: Schädigungstoleranz-Kennlinien des erfindungsgemäßen Zirkonoxidwerkstoffs und Verbundwerkstoffs im Vergleich zu Werkstoffen der Stand der Technik (Fig. 6)

Figur 6 zeigt die Restfestigkeitswerte nach unterschiedlichen Schädigungen (hier Vickershärte-Eindrücke mit unterschiedlichen Lasten von 3 bis 500 N) von unterschiedlichen Werkstoffsystemen, einem ZTA- (zirconia toughened alumina), einem Y-TZP- (Y-stabilisiertes polykristallines Zirkonoxid), einem erfindungsgemäßen Zirkonoxidwerkstoff Sm-TZP und einem erfindungsgemäßen Verbundwerkstoff (strontiumhexaaluminate toughened zirconia). Die geprüfte Eindrucklast ist logarithmisch in N auf der x-Achse gegen die Restfestigkeit in MPa auf der y-Achse aufgetragen.

Im Vergleich zu Werkstoffen aus dem Stand der Technik zeigt sich, dass die neuen erfindungsgemäßen Werkstoffe bei gleichbleibender Ausgangfestigkeit signifikant höhere Schädigungstoleranzen nach unterschiedlichen Schädigungslasten zeigen.

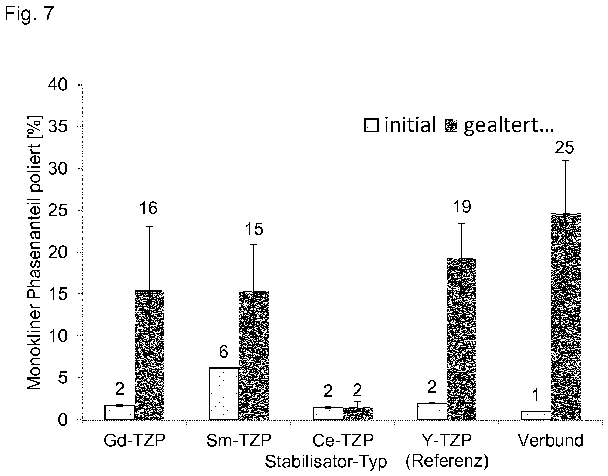

### Versuchsreihe 6: Hydrothermale Alterungsbeständigkeit in Abhängigkeit des chemischen Stabilisators (Fig. 7)

In Fig. 7 ist die hydrothermale Alterungsbeständigkeit der erfindungsgemäßen Zirkonoxidwerkstoffe in Abhängigkeit des verwendeten Stabilisators gezeigt. Dafür wurde an polierten Sinterformkörpern der monokline Phasenanteil mittels Röntgendiffraktometrie vor und nach Alterung gemessen. Die Auslagerung in hydrothermaler Atmosphäre erfolgt in einem Autoklaven bei 134°C und 2,2 bar Druck. Hierbei wurde ein Zyklus von 10 Stunden abgefahren.

Es hat sich überraschend gezeigt, dass die nicht erfindungsgemäße Variante mit CeO₂-Stabilisierung keine hydrothermale Alterung zeigt. Die erfindungsgemäßen Varianten mit Sm₂O₃- und Gd₂O₃-Stabilisierung zeigen eine geringfügige, aber signifikante Verbesserung der hydrothermalen Beständigkeit gegenüber dem Referenzwerkstoff Y-TZP.

Demnach weist Zirkonoxidwerkstoff gemäß einer besonders bevorzugten Ausführungsform der Erfindung eine verbesserte hydrothermale Alterungsbeständigkeit auf. Die verbesserte Alterungsbeständigkeit zeigt sich darin, dass der Anteil an monoklinem Zirkonoxid am Gesamtzirkonoxid-Gehalt nach Auslagerung in hydrothermaler Atmosphäre in einem Autoklaven bei 134°C und 2,2 bar Druck und einem Zyklus von 10 Stunden weniger als 17 Vol.-% und bevorzugt weniger als 10 Vol.-% und besonders bevorzugt weniger als 5 Vol.-% beträgt.

Im folgenden Abschnitt sind nochmals die Vorteile des erfindungsgemäßen Zirkonoxidwerkstoffs zusammengefasst.
- Herstellung des erfindungsgemäßen Zirkonoxidwerkstoffs und erfindungsgemäßer Sinterformkörper erfolgt mittels bekannter, konventioneller Keramiktechnologie
- 3-stufige Sinterung (Vorbrand, HIP, Weißbrand) möglich, daraus resultiert eine höhere Festigkeit
- keine hydrothermale Alterung durch die Verwendung von CeO₂ als chemische Stabilisierung
- schädigungsfreie, insbesondere mechanische Hartbearbeitung von dichtgesinterten oder teilgesinterten Zwischenprodukten möglich
- leichtere Hartbearbeitung durch geringere Werkstoffhärte (gleichbedeutend mit weniger Werkzeugverschleiß)
- geringere Härte, dadurch u.a. deutlich verringerter Abrieb des natürlichen Antagonisten im molaren Bereich
- Verwendung als vollanatomisches System möglich, d.h. Verblendung wird im molaren Bereich nicht benötigt, dadurch zusätzliche Kosteneinsparung für Patient und Verringerung der Gefahr des Abplatzens von Teilen der Verblendung (Chip-off)
- dentalgerechte Ästhetik
- Ausgleich zur fehlenden Resilienz (Dämpfung bzw. Federung des Zahns bei der Kaubewegung) im Falle einer kompletten Zahnrestauration mit Implantat, d.h. deutlich verringerter Spannungsaufbau bei Kaubewegung
- Zirkonoxidwerkstoff kann zur Herstellung von Blanks bzw. Blöcke für die CAD/CAM-Bearbeitung im vorgesinterten oder dichtgesinterten Zustand verwendet werden
- Verwendung der Sinterformkörper als Zahnersatz, zur Zahnrestauration (Brücken, Kronen, Inlays, Onlays), als Zahnwurzelstifte, Implantate, Abutments
- Verwendung zur Herstellung von Wirbelsäulen-Cages, Medizinischen Instrumenten u.a.

## Patentansprüche

1. Zirkonoxidwerkstoff, **dadurch gekennzeichnet, dass** der Gehalt an tetragonalem Zirkonoxid zwischen 94 und 96 Vol.-% liegt und die tetragonale Phase chemisch stabilisiert ist,
wobei als chemische Stabilisatoren Sm₂O₃ und/oder Gd₂O₃ oder Mischungen aus diesen Oxiden enthalten sind und der Anteil an Sm₂O₃ und Gd₂O₃ zwischen 1 und 5 Mol-%, jeweils relativ zum Zirkonoxidgehalt, liegt,
und
wobei eine zweite Hauptkomponente mit einem Volumengehalt zwischen 4 und 6 Vol.-% enthalten ist, die zu einem relativen Anteil von über 80 Vol.-% aus Strontiumaluminat oder Lanthanaluminat besteht.

2. Zirkonoxidwerkstoff nach einem der vorstehenden Ansprüche, **dadurch gekennzeichnet, dass** der Gesamtgehalt an chemischen Stabilisatoren < 15 Mol-% beträgt.

3. Zirkonoxidwerkstoff nach einem der vorstehenden Ansprüche, **dadurch gekennzeichnet, dass** das Zirkonoxid lösliche Bestandteile, insbesondere ein oder mehrere Verbindungen der Elemente Cr, Fe, Mg, Ca, Ti, Y, Sc, und/oder V enthält.

4. Zirkonoxidwerkstoff nach einem der vorstehenden Ansprüche, **dadurch gekennzeichnet, dass** die Zirkonoxidkristalle eine durchschnittliche Größe zwischen 0,1 und 1,5 µm aufweisen.

5. Zirkonoxidwerkstoff nach einem der vorstehenden Ansprüche, **dadurch gekennzeichnet, dass** die Härte des Zirkonoxidwerkstoffs kleiner als 1250 (HV10) ist.

6. Zirkonoxidwerkstoff nach einem der vorstehenden Ansprüche, **dadurch gekennzeichnet, dass** die Bruchfestigkeit ≥ 500 MPaist.

7. Zirkonoxidwerkstoff nach einem oder mehreren der vorstehenden Ansprüche, **dadurch gekennzeichnet, dass** die Schädigungstoleranz bzw. Restfestigkeit nach HV50-Eindruck > 500 MPa ist.

8. Zirkonoxidwerkstoff nach einem der vorstehenden Ansprüche, **dadurch gekennzeichnet, dass** der Werkstoff eine verbesserte hydrothermale Alterungsbeständigkeit aufweist, wobei der Anteil an monoklinem Zirkonoxid am Gesamtzirkonoxid-Gehalt nach Auslagerung in hydrothermaler Atmosphäre in einem Autoklaven bei 134°C und 2,2 bar Druck und einem Zyklus von 10 Stunden weniger als 17 Vol.-% und bevorzugt weniger als 10 Vol.-% und besonders bevorzugt weniger als 5 Vol.-% beträgt.

9. Verwendung eines Sinterformkörpers aus einem Zirkonoxidwerkstoff nach einem der Ansprüche 1 bis 8 als Zwischenprodukt, wobei der Sinterformkörper dichtgesintert oder teilgesintert ist, **dadurch gekennzeichnet, dass** das durch Sintern erzeugte Zwischenprodukt schädigungsfrei mechanisch bearbeitet werden kann.

10. Verwendung eines Sinterformkörpers aus einem Zirkonoxidwerkstoff nach einem der Ansprüche 1 bis 8 in der Dental- und Medizintechnik.

## Claims

1. Zirconium oxide material, **characterized in that** the content of tetragonal zirconium oxide is between 94 and 96 vol.% and the tetragonal phase is chemically stabilized,
Sm₂O₃ and/or Gd₂O₃ or mixtures consisting of these oxides being contained as chemical stabilizers and the proportion of Sm₂O₃ and Gd₂O₃ being between 1 and 5 mol%, relative to the zirconium oxide content in each case,
and
a second main component having a volume content between 4 and 6 vol.% being contained which consists of strontium aluminate or lanthanum aluminate in a relative proportion of over 80 vol.%.

2. Zirconium oxide material according to any of the preceding claims, **characterized in that** the total content of chemical stabilizers is < 15 mol%.

3. Zirconium oxide material according to any of the preceding claims, **characterized in that** the zirconium oxide contains soluble components, in particular one or more compounds of the elements Cr, Fe, Mg, Ca, Ti, Y, Sc, and/or V.

4. Zirconium oxide material according to any of the preceding claims, **characterized in that** the zirconium oxide crystals have an average size of between 0.1 and 1.5 µm.

5. Zirconium oxide material according to any of the preceding claims, **characterized in that** the hardness of the zirconium oxide material is less than 1250 (HV10).

6. Zirconium oxide material according to any of the preceding claims, **characterized in that** the breaking strength is ≥ 500 MPa.

7. Zirconium oxide material according to one or more of the preceding claims, **characterized in that** the damage tolerance or residual strength is > 500 MPa following a HV50 impression.

8. Zirconium oxide material according to any of the preceding claims, **characterized in that** the material has an improved hydrothermal aging resistance, the proportion of monoclinic zirconium oxide in the total zirconium oxide content being less than 17 vol.% and preferably less than 10 vol.% and particularly preferably less than 5 vol.% after age-hardening in a hydrothermal atmosphere in an autoclave at 134°C and 2.2 bar pressure and in a cycle of 10 hours.

9. Use of a sintered molded body consisting of a zirconium oxide material according to any of claims 1 to 8 as an intermediate product, the sintered molded body being densely sintered or partially sintered, **characterized in that** the intermediate product produced by sintering can be mechanically processed without being damaged.

10. Use of a sintered molded body consisting of a zirconium oxide material according to any of claims 1 to 8 in dental and medical technology.

## Revendications

1. Matériau à base d'oxyde de zirconium, **caractérisé en ce que** la teneur en oxyde de zirconium tétragonal est comprise entre 94 et 96 % en volume et que la phase tétragonale est stabilisée chimiquement,
du Sm₂O₃ et/ou du Gd₂O₃ ou des mélanges de ces oxydes étant présents en tant que stabilisants chimiques, la proportion de Sm₂O₃ et de Gd₂O₃ étant comprise entre 1 et 5 % en moles, dans chaque cas par rapport à la teneur en oxyde de zirconium, et
un second constituant principal étant présent à raison d'une teneur en volume comprise entre 4 et 6 % en volume, lequel est constitué, en proportion relative, de plus de 80 % en volume d'aluminate de strontium ou d'aluminate de lanthane.

2. Matériau à base d'oxyde de zirconium selon l'une des revendications précédentes, **caractérisé en ce que** la teneur totale en stabilisants chimiques est inférieure à 15 % en moles.

3. Matériau à base d'oxyde de zirconium selon l'une des revendications précédentes, **caractérisé en ce que** l'oxyde de zirconium contient des éléments solubles, en particulier un ou plusieurs composés des éléments Cr, Fe, Mg, Ca, Ti, Y, Sc et/ou V.

4. Matériau à base d'oxyde de zirconium selon l'une des revendications précédentes, **caractérisé en ce que** les cristaux d'oxyde de zirconium présentent une taille moyenne comprise entre 0,1 et 1,5 µm.

5. Matériau à base d'oxyde de zirconium selon l'une des revendications précédentes, **caractérisé en ce que** la dureté du matériau à base d'oxyde de zirconium est inférieure à 1 250 (HV10).

6. Matériau à base d'oxyde de zirconium selon l'une des revendications précédentes, **caractérisé en ce que** la résistance à la rupture est supérieure ou égale à 500 MPa.

7. Matériau à base d'oxyde de zirconium selon l'une ou plusieurs des revendications précédentes, **caractérisé en ce que** la tolérance à la dégradation ou résistance résiduelle après impression HV50 est supérieure à 500 MPa.

8. Matériau à base d'oxyde de zirconium selon l'une des revendications précédentes, **caractérisé en ce que** le matériau présente une résistance améliorée au vieillissement hydrothermique, la proportion d'oxyde de zirconium monoclinique dans la teneur totale en oxyde de zirconium après stockage en atmosphère hydrothermique dans un autoclave à une température de 134 °C et à une pression de 2,2 bar et après un cycle de 10 heures étant inférieure à 17 % en volume, et de préférence inférieure à 10 % en volume, et de manière particulièrement préférée inférieure à 5 % en volume.

9. Utilisation d'un corps moulé fritté constitué d'un matériau à base d'oxyde de zirconium selon l'une des revendications 1 à 8 en tant que produit intermédiaire, le corps moulé fritté étant densément fritté ou partiellement fritté, **caractérisée en ce que** le produit intermédiaire obtenu par frittage peut être usiné mécaniquement sans détérioration.

10. Utilisation d'un corps moulé fritté constitué d'un matériau à base d'oxyde de zirconium selon l'une des revendications 1 à 8 en technique dentaire et médicale.
